# EUROPEAN PATENT SPECIFICATION

(11) **EP 3 833 406 B1**
(45) Date of publication and mention of the grant of the patent: **10.12.2025**
(21) Application number: 19762486.9
(22) Date of filing: 31.07.2019
(51) Int. Cl.: A61L 2/22, B65G 53/46, G01F 11/24

(54) **METHOD FOR THE SANIFICATION OF AN APPARATUS FOR METERING POWDERS, AND CORRESPONDING METERING APPARATUS**
VERFAHREN ZUR DESINFEKTION EINER VORRICHTUNG ZUM DOSIEREN VON PULVERN UND ZUGEHÖRIGE DOSIERVORRICHTUNG
PROCÉDÉ DE DÉSINFECTION D'UN APPAREIL DE DOSAGE DE POUDRES ET APPAREIL DE DOSAGE CORRESPONDANT

(30) Priority: 09.08.2018 IT 201800007994
(43) Date of publication of application: 16.06.2021
(73) Proprietor: I.M.A. INDUSTRIA MACCHINE AUTOMATICHE S.p.A., 40064 Ozzano dell' Emilia (BO) (IT)
(72) Inventor: TREBBI, Claudio, 40064 OZZANO DELL'EMILIA (IT)
(74) Representative: Petraz, Gilberto Luigi
(86) International application number: PCT/IT2019/050179
(87) International publication number: WO 2020/031218

(56) References cited:
- WO-A1-2015/158429
- WO-A1-2017/203412
- DE-A1- 2 363 505
- DE-C- 707 791
- US-A- 4 205 711
- US-A- 5 174 088
- US-A- 5 339 999

## Description

### FIELD OF THE INVENTION

The present invention concerns a method for the sanification of an apparatus for metering powders and a corresponding apparatus for metering powders, in particular usable in the pharmaceutical, food, cosmetic or similar fields. The apparatus being able to perform a desired and defined metering of a certain quantity of powders inside suitable containers, bottles or suchlike.

### BACKGROUND OF THE INVENTION

As is known, there are apparatuses, or machines, for metering powders comprising a hopper into which a certain quantity of powder is poured, which is then transferred, for example by means of a shaker, to a metering chamber in which a metering device is positioned.

The treated powders can have the most varied granulometry and include powders so fine that they can move through a faint flow of air.

For example, the metering means can comprise a series of metering sectors into which the powder contained in the hopper is poured. Suitable rotation steps of the disc bring each of these metering sectors in correspondence with an outlet opening of the powder from the hopper.

The powder contained in each sector of the metering disk is then transferred in sequence to the assigned containers or receptacles.

In the state of the art, the movement of the powder can occur according to systems of the pneumatic or mechanical type, or a combination thereof.

The metering means are the most varied, although, hereafter, comprising all of them by way of example, we refer to a rotating disk with metering seatings and means to define on each occasion the volume to be metered. A disk of this type is described, for example, in US patent US-A-5.174.088 to move and form metered quantities of fibrous material, which in this case is chewing tobacco. However, the use of this disc in the tobacco sector means that it does not require the sanification and sterilization operations which are instead essential in the fields listed above.

However, it is known that the metering can occur both by volume (as in the general example case assumed here) and also by weight.

By way of example, as well as the characteristic of the volume, a vacuum system can be provided which, located selectively in connection with one or more metering sectors, can facilitate the suction and/or expulsion of the powder.

It is also known that the machines or apparatuses for metering powders which treat powders of the pharmaceutical or phytotherapeutic type need, either periodically or at each product change, suitable cleaning treatments, for example washing and/or sterilization treatments.

Carrying out the sanification of apparatuses for metering powders is very complex because the powder, especially where the granulometry is low or very low, can disperse in a diffused manner inside the metering apparatus. The powder can also deposit, in an indistinct and random manner, on the various elements of the apparatus, even penetrating inside them in positions that are difficult to access.

It is evident that to obtain an accurate and complete sanification of the metering apparatus and of the elements connected therein, the various components which in various ways come into contact with the powder, currently have to be disassembled by an operator and taken to suitable washing and sterilization areas, before being repositioned on the apparatus.

The sanitization operations or treatments are however very complex, above all because they require frequent manipulations of the metering apparatus and often the disassembly of different parts or components thereof. This involves downtimes, uncertainty about the level of cleanliness, risks to the health of the operator, and costs that are not always sustainable.

Furthermore, at the end of the operations, it is necessary to check that the seals of the machine, or of the metering apparatus, are functioning and it is also necessary, in the case of disassembly, to restore the adjustments.

A known solution to carry out sanification operations, including washing and/or sterilization, provides for example to transfer at least parts of the metering apparatus, for example the metering disk, on board an autoclave separate and independent of the metering apparatus.

This solution is somewhat complex since the parts of the apparatus to be cleaned do not remain positioned on board the machine and still need to be passed to and from another machine. In addition, with this solution, there are the machine times for the washing, as well as an additional and distant specific machine.

This increases the bulk and, at the same time, the costs and complexity of the plant for metering powders, as well as problems connected to the calibration and adjustment of the plant.

One example of an apparatus for metering powders and of a method for its cleaning is described in the international patent application WO-A1-2017/203412 in the name of the Applicant. This solution provides the use of a sonotrode, disposed in a washing manifold in communication with the operative chambers and configured to generate bubbles and micro-bubbles of air or vapor which, by imploding, exert a cleaning action on the components of the apparatus detaching powder residues from them. It is important to note that during the activation of the sonotrode, the apparatus and the washing manifold are hermetically insulated from the external environment and are maintained at an internal pressure which is higher than atmospheric pressure in order to facilitate the implosion of the air bubbles.

Other solutions known in the state of the art are described, for example, in documents US-A-4.205.711, DE-A1-2363505, WO-A1-2015/158429 and DE-C-707.791, in which pneumatic systems to transport materials are described, in some cases of loose materials, comprising a rotating disk provided with a plurality of chambers or pockets from which the materials are expelled thanks to the action of the vector fluid, for example compressed air. In documents US-A-4.205.711 and WO-A1-2015/158429 it is further specified that the pneumatic system can also perform a cleaning action on components of the system, in particular on the rotating disk. Since the rotating disk is not located in a chamber which, if necessary, can be hermetically separated from the external environment and/or from the pneumatic system to move the vector fluid, it is noted that these solutions known in the state of the art do not allow an effective cleaning action, with times and costs that are compatible with the need to achieve adequate productivity.

In another field of application, that is the food sector, a metering unit for filling cups with paste-form product is known in the art from US-A-5.339.999. The metering unit comprises reciprocating piston and its operation comprises successive sanitizing, production and cleaning phases.

The metering unit of US-A-5.339.999 is not suitable for metering in a precise and accurate manner powdery product. Moreover, this solution is devoid of a rotating disk with metering seatings and for this reason its productivity is very low.

Devices suitable to perform the washing and/or cleaning of the means for metering liquids are also known.

These devices are called CIP/SIP (acronym for Cleaning In Place/Sterilizing In Place). However, these systems cannot be used effectively in apparatuses for metering powders because they do not guarantee that an accurate and complete sanitization will be obtained in relation to every type and size of powder.

Other limitations and disadvantages of known conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to perfect an apparatus for metering powders that can overcome at least one of the disadvantages of the state of the art.

One purpose of the present invention is therefore to provide a method for the sanification of an apparatus for metering powders which is provided with a sanification system, for example washing and/or sterilization, which cooperates directly with the metering apparatus itself.

Such a solution guarantees an effective sanification of all the parts directly on board the metering apparatus, thus avoiding the disassembly or transfer of these parts toward another cleaning machine.

Such a solution limits or eliminates any manual operations, especially after carrying out the washing or sterilization, and therefore allows greater guarantees, absence of control and reactivation interventions, and lower costs.

Another purpose of the present invention is to perfect a complete and effective method for the sanification of an apparatus for metering powders which allows to carry out an accurate sanification quickly and automatically or semiautomatically.

Another purpose of the present invention is to provide an apparatus for metering powders which is compact, efficient and guarantees, on the one hand, an optimal metering of the powders, and on the other, effective sanification operations of the various parts of which it is made.

It is also a purpose of the present invention to provide an effective sanification system on board the metering machine even in the case of impalpable powders or powders which present or can present electrostatic charge problems.

Another purpose is to reduce downtimes, eliminate recalibrations and efficiency checks.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a method is provided for the sanification of an apparatus for metering powders which comprises a metering chamber and a volumetric rotating metering device for powders with vacuum/pressure cycles which is housed in the metering chamber in a predefined metering configuration, in which the metering device is suitable to deliver doses of a predetermined quantity of powder inside containers.

The sanification comprises a washing step and a subsequent sanitization/sterilization step of the metering device and/or possible components present in the metering chamber. The sanification is carried out after a powder metering cycle has been completed, where a powder metering cycle provides filling a predefined number of containers.

Here and throughout the present description, the term "sanification" refers to the entirety of processes to make hygienic or hygienically safe a plant or an apparatus, in particular intended to work products (for example pharmaceuticals) in a protected atmosphere; the term "sanitization" refers to the treatment, comprised in the sanification processes, aimed at reducing the bacterial or microbial load inside a plant or an apparatus, in particular intended to work products (for example pharmaceuticals) in a protected atmosphere; and the term "sterilization" refers to the treatment aimed at completely eliminating the bacterial or microbial load by means of sterilizing agents that effect the plant or apparatus, in particular intended to work products (for example pharmaceuticals) in a protected atmosphere, for a determined time period at high temperatures.

In one embodiment, the step of washing the metering device provides to transfer the latter into a washing chamber.

According to a characteristic aspect of the invention, the sanitization/sterilization step is carried out in situ by maintaining the metering device in the metering configuration as above, and comprises a first sub-step in which it is provided to bring the metering chamber to a pressure lower than the atmospheric one, and a subsequent second sub-step in which it is provided to introduce, through the inlet opening, a sanitizing/sterilizing fluid (vapor) inside the metering device, while the metering chamber is maintained at the pressure lower than the atmospheric one. During this sanitization/sterilization step it is therefore provided to create a vacuum inside the metering chamber, which is for example brought to a pressure equal to -0.7 bar.

Here and throughout the whole description the term "create a vacuum" refers to the operations with which it is possible to bring an environment that is closed, circumscribed and hermetically separable from the external environment - such as for example the metering chamber - into a condition of depression with respect to atmospheric pressure.

In this way, before carrying out a new metering cycle, manipulations or displacements of the metering device advantageously are not provided after the sanitization/sterilization step as above.

It should be noted that the sanitization/sterilization step of the metering device occurs after the metering device has been once again placed in the metering chamber, if it has previously been transferred into the washing chamber to execute the washing step.

In some embodiments, the sanitizing/sterilizing fluid can comprise a mixture of compounds, some of which are known in the state of the art, having antimicrobial and disinfectant properties.

In some embodiments, the sanitizing/sterilizing fluid can be a fluid, or a mixture of fluids, in the vapor phase.

In one embodiment, the sanitizing/sterilizing fluid can be a pressurized fluid.

Thanks to the sanification method described here, the metering device (and possibly further components disposed in the metering chamber) are sterilized in situ during the sanitization/sterilization step as above. In other words, the metering chamber is configured so as to be a pressurizable (or evidently de-pressurizable) chamber which itself acts as an autoclave.

One advantage of the sanification method according to the present invention is that, after the sanitization/sterilization step, the metering apparatus is immediately ready for use, that is, to deliver the powder in a new metering cycle, after the metering disk and the connected components have been sanitized/sterilized inside the metering chamber during the sanitization/sterilization step.

The fact that the sanitization/sterilization step occurs in situ - that is, directly in the metering chamber - is advantageous because it avoids any further manipulation, both manual and also automatic, of the components that have been sanitized/sterilized, manipulations that instead could compromise the sterile conditions reached during the sanitization/sterilization step.

In a possible implementation, the method comprises a step of washing the metering chamber in which it is provided to deliver a washing fluid into the metering chamber and flood the latter with the washing fluid as above. Thereafter, a step of draining the washing fluid with which the metering chamber was flooded is provided. In this implementation, the washing fluid can be delivered into the metering chamber after having transfered the metering device into the washing chamber, or, alternatively, when the metering device is located in the metering chamber.

The sanification method can include a drying step, possibly high-powered, by means of hot gaseous fluids.

In this drying step, means for controlling the ambient humidity or the transit walls can intervene.

A further purpose of the present invention is an apparatus for metering powders comprising a metering chamber, a device to meter powders housed in the metering chamber in a predefined metering configuration in which the metering device meters the powders, and at least one unit to feed the powders to be metered toward this metering device.

The metering device is configured to deliver a suitable quantity of powders to one or more containers located downstream of the metering device.

According to a characteristic aspect of the invention, the metering apparatus comprises at least one unit to deliver at least one sanitizing/sterilizing fluid or a washing fluid integrated in the metering apparatus, or directly associated therewith, and configured to send the fluid at least into the metering chamber.

In this way, it is possible to achieve sanification of the components disposed in the metering chamber, in particular the metering device, directly in the chamber, without needing to extract them from the metering apparatus.

According to a variant, this solution can comprise means to dry the metering chamber and the connected devices.

According to a further variant, means are provided to control the possible humidity, present and/or residual, in the metering device and/or in the device for the entry and possible fluidization of the powders and/or in the metering chamber, that is, throughout the whole travel of the powders as they are metered or released into the designated container.

According to a further variant, means are provided to dry, partially or totally, the path along which the powders are metered or released into the designated container.

The drying means can be means that heat the passages, that is, a hot gaseous fluid which travels through all, or part, of the path along which the powders are metered or released into the designated container.

According to another variant, inside the metering device are provided pipes configured so that a thermal vector fluid, for example a hot liquid fluid, passes through them in order to thermally insulate the metering chamber to prevent unwanted heat dissipation.

According to a further variant, the path along which the powders are metered and released, before the metering step, is autonomous and independent from the metering device which instead can cooperate with at least one delivery unit of the sterilizing or washing fluid.

Another variant is to provide that the metering device is engaged with a circulating or temporally stationary mass of a washing fluid which submerges the metering device itself.

A further variant is to use, at least for a certain part of the sanitization cycle, a gaseous sterilizing or washing fluid.

It is also a variant to use, at least for a part of the sanitization cycle, a liquid sterilizing or washing fluid, be it cold or at a controlled temperature.

In one embodiment, a washing chamber is provided in which at least one cleaning device is installed, configured to house at least the metering device, at least in a washing step thereof.

In one embodiment, the washing chamber and the metering chamber can be integrated into a single monobloc containing body. In this case, the metering chamber and the washing chamber are configured to be put in communication by means of a passage opening which can be selectively closed by a mobile division so as to hermetically separate them.

In one embodiment, the feed unit of the powders and the fluid delivery units can comprise pipes in fluid communication with a single collector located upstream of the metering chamber.

In one embodiment, the feed unit of the powders and the fluid delivery units comprise one or more transit pipes shared by the powders and the fluid, which are alternately passed through by the powders or the fluid in the different metering and sanitization steps.

According to another aspect of the present invention, a method is provided for the functioning of an apparatus for metering powders comprising a powder metering cycle in which it is provided to deliver an appropriate quantity of powder into one or more containers by means of a volumetric rotating metering device for powders with vacuum/pressure cycles, and a sanification cycle that comprises a washing step and a sanitization/sterilization step of the metering device and possibly of other components of the metering apparatus subjected to sanification.

According to this aspect of the present invention, it is provided that the sanitization/sterilization step is carried out in situ maintaining the metering device in the metering configuration as above inside the metering chamber so that, before carrying out a new powder metering cycle, no manipulations or displacements of the metering device are provided after the sanitization/sterilization step. This last step comprises a first sub-step in which it is provided to bring the metering chamber to a pressure lower than the atmospheric one, and a subsequent second sub-step in which it is provided to introduce, through the inlet opening, a sanitizing/sterilizing fluid (vapor) inside the metering device, while the metering chamber is kept at the pressure lower than the atmospheric one.

These and other aspects, characteristics and advantages of the present disclosure will be better understood with reference to the following description, drawings and attached claims. The drawings, which are integrated and form part of the present description, show some embodiments of the present invention.

### BRIEF DESCRIPTION OF THE DRAWINGS

These and other characteristics of the present invention will become apparent from the following description of some embodiments, given as a non-restrictive example with reference to the attached drawings wherein:

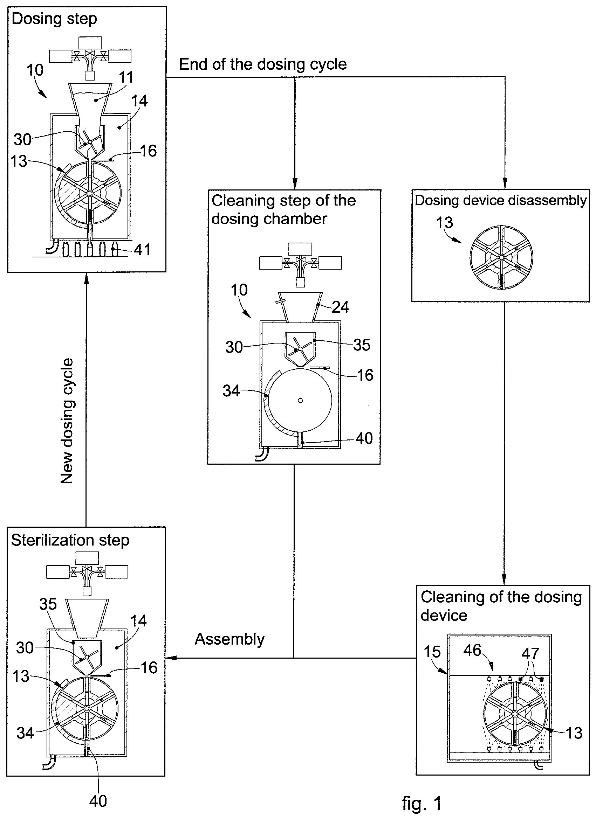

- fig. 1 is a schematic view of a block diagram showing an embodiment of a sanification method according to the present invention, in which the metering apparatus is shown in different operative steps;
- fig. 2 is a longitudinal, partial and schematic section view of an apparatus for metering powders according to the present invention, shown during a powder metering cycle;
- fig. 3 is a longitudinal, partial and schematic section view of a variant embodiment of the apparatus of fig. 2;
- fig. 4 is a longitudinal, schematic section view of a washing chamber associated with the metering apparatus of fig. 1;
- fig. 5 is a schematic three-dimensional view of an embodiment of an apparatus for metering powders according to the present invention;
- fig. 6 is a longitudinal, schematic section view of the metering apparatus of fig. 5 shown in a washing step of the corresponding metering device;
- fig. 7 is a longitudinal, partial and schematic section view of another variant embodiment of the apparatus of fig. 2;
- fig. 8 is a longitudinal, partial and schematic section view of a further variant embodiment of the apparatus of fig. 2.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to example embodiments of the present invention. For example, the characteristics shown or described insomuch as they are part of one embodiment can be adopted on, or in association with, other embodiments to produce another embodiment. It is understood that the present invention shall include all such modifications and variants.

With reference to the attached drawings, an apparatus 10 for metering powders 11, according to the present invention, comprises a metering chamber 14 in which a metering device 13 is housed, comprising for example a metering disc, or suchlike.

The following example provides, by way of example, a specific metering disc, in particular a volumetric rotating metering device for powders with vacuum/pressure cycles.

In one embodiment, the metering disk comprises a plurality of metering seatings 36, each intended to receive the volume of powder **11** metered that has to be transferred from the metering device 13.

In some embodiments, the metering chamber 14 can be opened or closed by means of a corresponding hatch 31, possibly hermetic, for normal maintenance, calibration, etc. operations.

Suitable sealing elements can be provided, not shown, associated with the hatch 31 so that the metering chamber 14 is a closed chamber which can be pressurized under certain operative conditions, for example during the sterilization step.

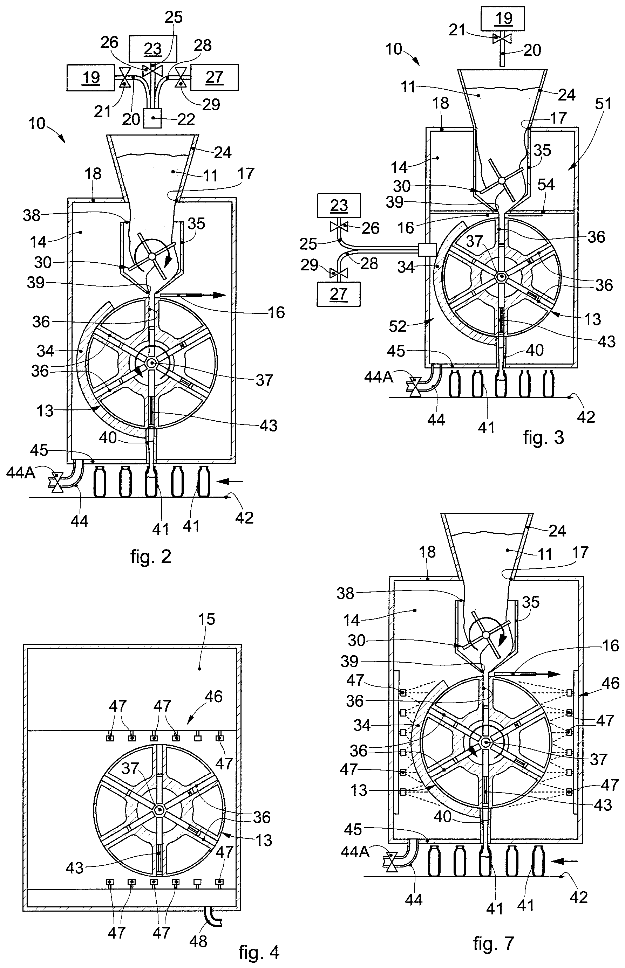

According to a variant, the metering apparatus 10 also comprises a washing chamber 15 (fig. 4) separated from the metering chamber 14, into which the metering device 13 can be transferred in order to undergo one or more cleaning treatments, for example washing and/or rinsing.

In the washing chamber 15, nozzles 47 of a cleaning device 46, and a possible drain pipe 48 can be provided.

The washing chamber 15 can be opened or closed by means of a corresponding hatch 32, for the normal tuning, control, etc. operations.

In one embodiment, the hatch 32 is a hatch of the type with a double wall, with a double packing, commonly used in metering devices with protected atmosphere known in the state of the art.

In one embodiment, the washing chamber 15 of fig. 3 is separated from the metering chamber 14, for example being disposed in its vicinity but so that it is not directly connected to the metering chamber 14, shown by way of example in figs. 2 and 3.

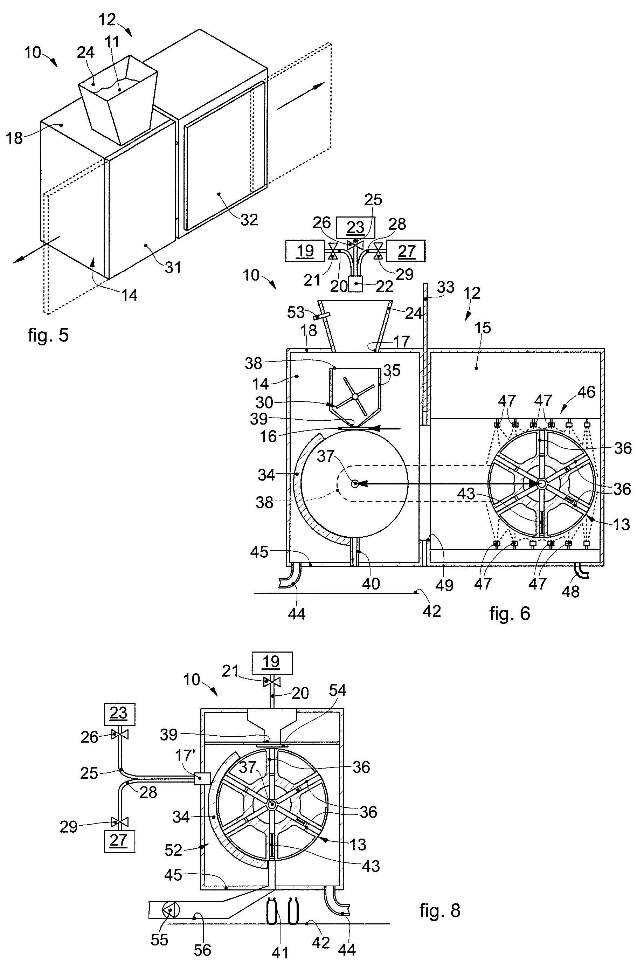

In an alternative embodiment, shown in figs. 5 and 6, the metering chamber 14 and the washing chamber 15 are adjacent and normally separated.

In this embodiment, the metering chamber 14 and the washing chamber 15 are integrated in a single containing body 12, which defines a single monobloc or monolith of the metering apparatus 10.

However, the metering chamber 14 and the washing chamber 15 can be put in communication with each other by, for example, mobile dividing means 33 which condition a passage opening 49, see also fig. 6.

In this variant, in order to reduce the downtimes caused by washing and sterilization, two or more metering devices 13 can be provided, so that during the washing and sterilization step of one, the other can operate.

Means not shown provide to change the metering devices 13.

It is possible to provide, for example, that the metering device 13 is provided with means to be attach to and detached from the metering chamber 14, and that between the metering chamber 14 and the washing chamber 15 transfer means 38 are provided, suitable to allow a passage of the metering device 13 from the metering chamber 14 to the washing chamber 15, and vice versa.

In some embodiments, the transfer means can be of a type known in the state of the art. For example, the transfer means can be configured as linear guides along which the metering device 13 is moved, in particular by means of suitable movement means of the mechanical type.

In some embodiments, the metering apparatus 10 comprises drying means, of a type known in the state of the art and not shown, which are associated with the metering device 13 and/or the metering chamber 14 and/or the washing chamber 15.

The powder 11 to be metered enters the metering chamber 14 through an inlet opening 17, disposed for example on the upper wall 18 of the containing body 12.

An introduction hopper 24 can be associated with the inlet opening 17 in order to receive the powder 11.

Downstream of the inlet opening 17 there is a hopper 35 to receive and release the powder 11. The hopper 35 can cooperate with a shaking device 30 which, according to a variant, can also temporarily close an outlet opening 39 from which the powder 11 exiting the hopper 35 can reach the metering device 13.

It should be noted that the shaking device 30 acts as fluidization means of the powders.

The shaking device 30 therefore has the function of preventing the powder 11 to be metered from agglomerating, and possibly keeping the walls of the hopper 35 clean.

The shaking device 30 and the metering device 13 can be separated by a door 16, for example sliding, and possibly sealed, which allows to open or close the passage of the powder 11 to be metered toward the metering device 13.

According to a further variant (fig. 3), the introduction hopper 24 and the hopper 35 are located in continuity and the environment thus created can be with a controlled pressure and/or temperature and/or atmospheric composition according to one or more of these factors.

According to a further variant, the end part of the shaker 30 is configured to exert a slight pressure (desired) toward the powder to be introduced into the metering device 13, in order to guarantee the complete filling of its metering sectors 36.

In one embodiment, the metering apparatus 10 comprises at least one humidity sensor 53 which allows to detect the presence of humidity which can be caused by the environmental situation or by cleaning and/or sterilization residues.

In some embodiments, the humidity sensor 53 can be associated with different components of the metering apparatus 10. For example, the humidity sensor 53 can be associated with the introduction hopper 24 (fig. 6).

A feed unit 19 of powder is provided upstream of the inlet opening 17, suitable to transfer the powder 11 to be metered to said inlet opening 17 by means of a pipe 20.

The pipe 20 can be provided with a regulating valve 21, suitable to regulate the flow of powder 11 toward the inlet opening 17.

The pipe 20 and the introduction hopper 24 can be in a closed circuit and with a controlled atmosphere.

In one embodiment, shown in fig. 2, upstream of the inlet opening 17 a first delivery unit 23 of a sanitizing/sterilizing fluid is also provided, suitable to deliver the fluid toward the inlet opening 17 by means of a corresponding pipe 25.

The pipe 25 can be provided with a regulating valve 26 to regulate the flow of the first sanitizing/sterilizing fluid toward the inlet opening 17.

Preferably, the pipe 25 transfers the sanitizing/sterilizing fluid toward a collector 22, which pours it through the hopper 35 into the metering chamber 14.

In one embodiment, upstream of the inlet opening 17 a second delivery unit 27 of a washing fluid is also provided, suitable to deliver the washing fluid inside the metering chamber 14, in particular toward the inlet opening 17 by means of a corresponding pipe 28.

The pipe 28 can be provided with a regulating valve 29 to regulate the flow of the washing fluid toward the inlet opening 17.

As an alternative to what we have just described and shown, it is possible to provide a single delivery unit suitable to supply a fluid that can perform both the washing and sanitization/sterilization, or it is also possible to provide a single delivery unit suitable to supply, alternatively, a first cleaning fluid, for example a washing fluid, or a second cleaning fluid, for example sanitizing/sterilizing fluid.

The metering seatings 36 of the metering device 13 are located radially with respect to an axis 37 around which it can rotate, by means of suitable drive means, not shown.

The rotation of the metering device 13 allows to bring the metering seatings 36, on each occasion, facing toward the shaking device 30 which delivers the desired quantity of powder 11 inside each metering seating 36.

Regulating the quantity of powder to be metered can occur by means of suitable regulating systems of the type known in the state of the art, which, for example, position at the desired volume a plunger 43 with respect to the entrance mouth of the metering seating 36.

In one embodiment, downstream of the metering device 13 is provided at least one outlet pipe 40 of the powder 11 from each of the metering seatings 36 toward a container 41 into which to pour the metered powder 11.

In an alternative embodiment, not shown, the metering device does not have the outlet pipe 40. In this case, the powder 11 exiting the metering seatings 36 is poured directly into the containers 41. In this embodiment, in order to avoid unwanted dispersions of the powders, the bottom wall 45 of the metering chamber 14 is substantially tangent to the metering device 13.

The containers 41 can be moved by a conveyor belt 42, or suchlike, suitable to bring a sequence of containers 41 to be filled below said outlet pipe 40 and therefore the metering device 13.

In the embodiments shown, the conveyor belt 42 is disposed so as to advance the containers 41 in a direction of advance substantially orthogonal to the axis 37.

In an alternative embodiment, not shown, the conveyor belt 42 can be disposed so as to advance the containers 41 in a direction of advance substantially parallel to the axis 37.

The sequence of presentation of the containers 41 is coordinated with the desired positioning of the metering seating 36 ready for delivery.

The entrance mouth of the metering seating 36, in the case shown, cooperates with a containing wall 34 in order to prevent the powder present in the metering seating 36 from exiting and changing the dose.

The metering seating 36 can cooperate with fluid delivery nozzles that flow into it and allow to discharge all the powder 11 and also clean the seating 36 itself. The sanitizing/sterilizing or washing fluid can be delivered tangentially to the metering seating 36 and with a desired inclination in order to guarantee the best cleaning of the sector itself.

The metering chamber 14 in which the metering device 13 is disposed is provided with a drain pipe 44, preferably made on the bottom wall 45 thereof. The drain pipe 44 can be opened or closed automatically by suitable opening/closing means. By way of example, in the embodiments of figs. 2, 3 and 7, these means are schematized as a shut-off valve 44A of a type known in the state of the art.

According to a variant, shown in fig. 3, in cooperation with the outlet opening 39 of the hopper 35, or rather with the door 16, there is a dividing wall 54 which divides the metering chamber 14 into two parts, defining two chambers 51, 52.

By doing so, it is possible to insulate the metering chamber 14 in which the metering device 13 is located, which in the meantime has positioned itself to close the outlet pipe 40, that is, the outlet pipe 40 has been divided, in order to flood the chamber 52 with the cleaning liquid or fluid, and possibly with the sanitizing/sterilizing liquid or fluid. It should be noted that in this variant, while the powders are fed from above into the hopper 35, coming from the feed unit 19, the various fluids coming from the respective delivery units 23, 27, are introduced into the metering chamber 14 through a respective inlet opening 17', different from the inlet opening 17 of the powders. In the example shown, this respective inlet opening 17' has been shown on one of the lateral walls of the metering chamber 14, but - as the person of skill in the art can easily realize - it can naturally be placed in any other suitable position whatsoever, such as for example on a wall at the top of the chamber.

Once the sanitization/sterilization step and/or the washing step have ended, the drain pipe 44 is opened and the chamber 52 is emptied of the corresponding fluid used in these steps.

According to a variant, drying fluids, such as for example air or hot gas, are introduced into the chamber 52 to dry the whole.

Means to control the ambient humidity can be activated in order to check the progression of the drying and the completion of the drying. Similarly, means to control humidity can be provided in relation to the metering seatings 36 and/or to the outlet pipe 40.

According to a further variant embodiment, shown in fig. 7, the cleaning device 46 is installed in the metering chamber 14, which also acts as a washing chamber. In this variant embodiment, the metering apparatus 10 doesn't have a dedicated washing chamber and the washing step as above is carried out inside the metering chamber according to the modes described above. In a possible implementation, it is provided that the nozzles 47 of the cleaning device 46 deliver the washing fluid into the metering chamber 14, directing it in particular toward the metering device 13, in order to thoroughly clean the latter.

The metering apparatus 10 comprises a control and command unit, not shown, which automatically commands both the step of metering the powder **11,** and also the washing and/or sanitization/sterilization steps described above.

Furthermore, the control and command unit can also command the opening and closing of the mobile division 33 and the transfer of the metering device 13, and possibly the exchange of the two or more metering devices 13. In particular, the control and command unit can be set to move the mobile division 33 and the metering device 13 after each metering cycle, or after a certain predetermined number of metering cycles.

With reference to the drawings, we will now describe an example of an embodiment of a functioning method of the metering apparatus 10.

The metering apparatus 10 performs a metering cycle as described above.

Subsequently, at the end of the metering cycle, the metering device 13 is disassembled from the predefined metering configuration in which it meters the powders and is transferred from the metering chamber 14 to a washing chamber 15. For example, the transfer of the metering device 13 can be performed manually by an operator.

At this point, the method according to the invention provides a sanification cycle.

The sanification cycle comprises a washing step, as described above.

The washing step comprises a step of washing the metering device 13 inside a washing chamber 15, separate from the metering chamber 14, in which the cleaning device 46 is present.

The washing step further comprises a step of washing the metering chamber 14, and the components present therein, such as for example the door 16, the shaking device 30, the containing wall 34, the hopper 35 and the outlet pipe 40.

After that, it is provided to mount the metering device 13 in the metering chamber 14, which returns to the metering configuration as above.

Subsequently, the sanification cycle provides to sanitize/sterilize in situ the metering device 13, the metering chamber 14 and possibly the components present therein which have been previously listed.

Before the sanitization/sterilization step begins, it is provided to hermetically insulate the metering chamber 14. To do this, with reference by way of example to the version of fig. 8, it is necessary to close the valves 26, 29, 44A and the dividing wall 54. In this way, the metering chamber 14 in which the metering device 13 is located is hermetically separated from the external environment. After this, it is provided to activate the vacuum pump 55 which is in communication with the metering chamber 14 by means of the pipe 56 to create a vacuum in the metering chamber 14. Subsequently, it is provided to open the regulating valve 26 to introduce into the metering chamber 14 the first sterilizing fluid coming from the first delivery unit 23.

According to embodiments provided here, the activation of the vacuum pump 55 can bring the metering chamber to a pressure comprised between -1 bar and - 0.5 bar, in particular equal to about -0.7 bar. It has been verified that these pressure values are optimal to guarantee an efficient sterilization cycle, optimizing at the same time both the energy consumption to maintain the vacuum, and also the quantity of sterilizing fluid delivered.

It should also be noted that the sanitization/sterilization step occurs for a predefined period of time in delivery cycles of the sanitizing/sterilizing fluid inside the metering chamber 14, and therefore inside the metering device 13 located therein, alternated with overpressure cycles in which the pressure inside the metering chamber 14 is increased to a value higher than atmospheric pressure for a predefined period of time. In particular, the overpressure cycles provide to bring the metering chamber 14 to a pressure comprised between 1.2 and 1.8 bar, in particular equal to about 1.5 bar.

According to one embodiment, the method can provide to suitably move the mobile members of the metering device 13 during the sanitization/sterilization step. For example, during this step it can be provided to move the plungers 43 each within the respective metering seating 36, in particular with a slow movement coordinated with the rotation of the metering device 13. In this way, it is possible to facilitate the entry and permanence of the sanitizing/sterilizing fluid even in the most remote and difficult to reach positions of the device, facilitating its circulation inside the metering device 13 thanks to the movement of the mobile components of the device itself.

Once the sanitization/sterilization step has ended, carried out in the manner described above, the metering apparatus 10 can start a new powder **11** metering cycle, possibly with powders to be metered which differ in type from those previously metered.

It is also clear that, although the present invention has been described with reference to some specific examples, a person of skill in the art shall certainly be able to achieve many other equivalent forms of an apparatus for metering powders and of a method for the sanification of this apparatus, and of a method for its functioning, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Method for the sanification of an apparatus for metering powders (11), comprising a metering chamber (14) and a volumetric rotating metering device (13) for powders (11) with vacuum/pressure cycles, housed in said metering chamber (14) in a predefined metering configuration, in which said metering apparatus comprises a powder inlet opening (17) and an outlet pipe (40) for powder (11), said metering device (13) being suitable to deliver doses of a predetermined quantity of powder (11) through said outlet pipe (40) inside containers (41), the filling of a predefined number of containers (41) defining one powder metering cycle; said sanification being carried out after a powder metering cycle has been completed and comprising a washing step to wash said metering device (13) and a subsequent sanitization/sterilization step carried out in situ to sanitize/sterilize said metering device (13) that is maintained in said metering configuration inside said metering chamber (14) during the sanitization/sterilization step so that, before carrying out a new powder metering cycle, no manipulation or displacement of said metering device (13) is provided after said sanitization/sterilization step, said method being **characterized in that** said sanitization/sterilization step comprises a first sub-step in which said metering chamber (14) is brought to a pressure lower than atmospheric pressure and a subsequent sub-step in which, through said inlet opening (17; 17'), a sanitizing/sterilizing fluid (vapor) is introduced inside said metering device (13), while said metering chamber (14) is maintained at said pressure lower than atmospheric pressure.

2. Method as in claim 1, **characterized in that** said washing step provides to introduce a washing fluid inside said metering chamber (14) through said inlet opening (17; 17') and to flood said metering chamber (14) with said washing fluid, and is followed by a subsequent step of draining the washing fluid from said metering chamber (14).

3. Method as in claim 1, **characterized in that** said washing step provides to leave said metering device (13) in said predefined metering configuration inside said metering chamber (14), in said washing step it being provided to drive a cleaning device (46) installed in said metering chamber (14), able to deliver a washing fluid directed in particular toward said metering device (13).

4. Method as in any claim hereinbefore, **characterized in that** during said first sub-step in which said metering chamber (14) is brought to a pressure lower than atmospheric pressure it is provided to create a vacuum inside the metering chamber (14) by means of a vacuum pump (55) connected to the outlet pipe (40) of the powder (11), the vacuum being created after the powder inlet opening (17) has been closed.

5. Method as in any claim hereinbefore, **characterized in that** in said first sub-step it is provided to bring said metering chamber (14) to a pressure comprised between -1 bar and -0.5 bar, in particular equal to about -0.7 bar.

6. Method as in any claim hereinbefore, **characterized in that** said sanitization/sterilization step occurs for a predefined period of time in delivery cycles of the sanitizing/sterilizing fluid inside said metering chamber (14), and therefore inside said metering device (13), alternating with overpressure cycles in which the pressure within the metering chamber (14) is increased to a value higher than atmospheric pressure for a predefined period of time.

7. Method as in claim 6, **characterized in that** said overpressure cycles provide to take the metering chamber (14) to a pressure comprised between 1.2 and 1.8 bar, in particular equal to about 1.5 bar.

8. Method as in any claim hereinbefore, **characterized in that,** after said sanitization/sterilization step, it comprises a drying step to dry said metering chamber (14) and said metering device (13).

9. Method for the functioning of an apparatus for metering powders (11), comprising a metering chamber (14) and a volumetric rotating metering device (13) for powders (11) with vacuum/pressure cycles, housed in said metering chamber (14) in a predefined metering configuration in which said metering device comprises a powder inlet opening (17) and a powder outlet pipe (40), and said metering device (13) is also suitable to deliver doses of a predetermined quantity of powder (11) through said outlet pipe (40) inside containers (41), the filling of a predefined number of containers (41) defining one powder metering cycle, said method comprising performing a metering cycle of the powders (11) by means of the delivery of said doses of powder (11) inside said containers (41), and a subsequent sanification cycle which comprises a washing step to wash said metering device (13) and a subsequent sanitization/sterilization step to sanitize/sterilize said metering device (13) according to the method of any of claims 1 to 8.

10. Apparatus for metering powders, comprising a metering chamber (14) and a volumetric metering device (13) for powders (11) with vacuum/pressure cycles housed in said metering chamber (14) in a predefined metering configuration in which said apparatus comprises a powder inlet opening (17) and a powder outlet pipe (40), and said metering device (13) is suitable to deliver quantities of a predetermined quantity of powder (11) through said outlet pipe (40) inside containers (41); said apparatus also comprises a feed unit (19) of the powders (11) to be fed to said metering device (13) through said inlet opening (17), a pump (55) communicating with said metering chamber (14) and a delivery unit (23) to deliver a sanitizing/sterilizing fluid directly associated with the metering apparatus, said delivery unit (23) to deliver a sanitizing/sterilizing fluid being configured to deliver said sanitizing/sterilizing fluid inside said metering device (13) through said inlet opening (17; 17'), maintaining said metering device (13) disposed in said metering configuration inside said metering chamber (14), said apparatus being **characterized in that** said pump is a vacuum pump (55) that is configured to bring said metering chamber (14) to a pressure lower than atmospheric pressure before said sanitizing/sterilizing fluid is delivered and **in that** said metering device (13) is a volumetric rotating metering device (13).

11. Metering apparatus as in claim 10, **characterized in that** it comprises a washing chamber (15) in which at least one cleaning device (46) is installed to deliver a washing fluid, and configured to house at least the metering device (13) during a washing step, said metering chamber (14) and said washing chamber (15) being adjacent and normally separated, and put in communication with each other by means of mobile dividing means (33) which intercept a passage opening (49).

12. Metering apparatus as in claim 10, **characterized in that** it comprises at least one cleaning device (46) installed in said metering chamber (14) and configured to deliver a washing fluid inside said metering chamber (14) during a washing step.

13. Metering apparatus as in claim 10, **characterized in that** it comprises a delivery unit (27) of a washing fluid directly associated with the metering apparatus.

14. Metering apparatus as in any claim from 10 to 13, **characterized in that** said feed unit (19) of the powders and said delivery unit (23) of a sanitizing/sterilizing fluid comprise pipes (20, 25) in fluidic communication with a single collector (22).

## Patentansprüche

1. Verfahren zur Desinfektion einer Vorrichtung zum Dosieren von Pulvern (11), die eine Dosierkammer (14) und eine rotierende Volumen-Dosiervorrichtung (13) für Pulver (11) mit Vakuum-/Druckzyklen umfasst, die in einer vordefinierten Dosierkonfiguration in der Dosierkammer (14) untergebracht ist, wobei die Dosiervorrichtung eine Pulvereinlassöffnung (17) und ein Auslassrohr (40) für Pulver (11) umfasst, wobei die Dosiervorrichtung (13) geeignet ist, Dosen einer vorbestimmten Menge Pulver (11) durch das Auslassrohr (40) in Behälter (41) abzugeben, wobei das Befüllen einer vordefinierten Anzahl von Behältern (41) einen Pulverdosierzyklus definiert; wobei die Desinfektion nach Abschluss eines Pulverdosierzyklus durchgeführt wird und einen Waschschritt zum Waschen der Dosiervorrichtung (13) und einen anschließenden in situ durchgeführten Desinfektions-/Sterilisationsschritt zum Desinfizieren/Sterilisieren der Dosiervorrichtung (13) umfasst, während die Dosiervorrichtung (13) in der Dosierkonfiguration innerhalb der Dosierkammer (14) während des Desinfektions-/Sterilisationsschritts gehalten wird, sodass vor dem Durchführen eines neuen Pulverdosierzyklus keine Manipulation oder Verschiebung der Dosiervorrichtung (13) nach dem Desinfektions-/Sterilisationsschritts vorgesehen ist, wobei das Verfahren **dadurch gekennzeichnet ist, dass** der Desinfektions-/Sterilisationsschritt einen ersten Unterschritt umfasst, in dem die Dosierkammer (14) auf einen Druck gebracht wird, der niedriger ist als der atmosphärische Druck, und einen anschließenden Unterschritt, in dem durch die Einlassöffnung (17; 17') ein Desinfektions-/Sterilisationsfluid (Dampf) in die Dosiervorrichtung (13) eingeführt wird, während die Dosierkammer (14) auf einem Druck gehalten wird, der niedriger ist als der atmosphärische Druck.

2. Verfahren nach Anspruch 1, **dadurch gekennzeichnet, dass** der Waschschritt das Einführen eines Waschfluids in die Dosierkammer (14) durch die Einlassöffnung (17; 17') und das Fluten der Dosierkammer (14) mit dem Waschfluid vorsieht, worauf ein anschließender Schritt des Ablassens des Waschfluids aus der Dosierkammer (14) folgt.

3. Verfahren nach Anspruch 1, **dadurch gekennzeichnet, dass** der Waschschritt vorsieht, die Dosiervorrichtung (13) in der vordefinierten Dosierkonfiguration in der Dosierkammer (14) zu belassen, wobei in dem Waschschritt vorgesehen ist, eine in der Dosierkammer (14) installierte Reinigungsvorrichtung (46) anzutreiben, die in der Lage ist, ein insbesondere auf die Dosiervorrichtung (13) gerichtetes Waschfluid abzugeben.

4. Verfahren nach einem der vorhergehenden Ansprüche, **dadurch gekennzeichnet, dass** während des ersten Unterschritts, in dem die Dosierkammer (14) auf einen Druck unterhalb des atmosphärischen Drucks gebracht wird, vorgesehen ist, in der Dosierkammer (14) mittels einer an das Auslassrohr (40) für das Pulver (11) angeschlossenen Vakuumpumpe (55) ein Vakuum zu erzeugen, wobei das Vakuum erzeugt wird, nachdem die Pulvereinlassöffnung (17) geschlossen wurde.

5. Verfahren nach einem der vorhergehenden Ansprüche, **dadurch gekennzeichnet, dass** im ersten Unterschritt vorgesehen ist, die Dosierkammer (14) auf einen Druck zwischen -1 bar und -0,5 bar, insbesondere etwa -0,7 bar, zu bringen.

6. Verfahren nach einem der vorhergehenden Ansprüche, **dadurch gekennzeichnet, dass** der Desinfektions-/Sterilisationsschritt für einen vordefinierten Zeitraum in Zufuhrzyklen des Desinfektions-/Sterilisationsfluids in der Dosierkammer (14) und somit in der Dosiervorrichtung (13) stattfindet, abwechselnd mit Überdruckzyklen, in denen der Druck in der Dosierkammer (14) für einen vordefinierten Zeitraum auf einen Wert erhöht wird, der über dem atmosphärischen Druck liegt.

7. Verfahren nach Anspruch 6, **dadurch gekennzeichnet, dass** die Überdruckzyklen dafür sorgen, dass die Dosierkammer (14) auf einen Druck zwischen 1,2 und 1,8 bar, insbesondere etwa 1,5 bar, gebracht wird.

8. Verfahren nach einem der vorhergehenden Ansprüche, **dadurch gekennzeichnet, dass** es nach dem Desinfektions-/Sterilisationsschritt einen Trocknungsschritt zum Trocknen der Dosierkammer (14) und der Dosiervorrichtung (13) umfasst.

9. Verfahren zum Betreiben einer Vorrichtung zum Dosieren von Pulvern (11), umfassend eine Dosierkammer (14) und eine rotierende Volumen-Dosiervorrichtung (13) für Pulver (11) mit Vakuum-/Druckzyklen, die in der Dosierkammer (14) in einer vordefinierten Dosierkonfiguration untergebracht ist, in der die Dosiervorrichtung eine Pulvereinlassöffnung (17) und ein Pulverauslassrohr (40) umfasst, und die Dosiervorrichtung (13) auch geeignet ist, Dosen einer vorbestimmten Menge Pulver (11) durch das Auslassrohr (40) in Behälter (41) abzugeben, wobei das Befüllen einer vordefinierten Anzahl von Behältern (41) einen Pulverdosierzyklus definiert, wobei das Verfahren das Durchführen eines Dosierzyklus der Pulver (11) mittels der Abgabe der Pulverdosen (11) in die Behälter (41) und einen anschließenden Desinfektionszyklus umfasst, der einen Waschschritt zum Waschen der Dosiervorrichtung (13) und einen anschließenden Desinfektions-/Sterilisationsschritt zum Desinfizieren/Sterilisieren der Dosiervorrichtung (13) gemäß einem Verfahren nach einem der Ansprüche 1 bis 8 umfasst.

10. Vorrichtung zum Dosieren von Pulvern, umfassend eine Dosierkammer (14) und eine Volumen-Dosiervorrichtung (13) für Pulver (11) mit Vakuum-/Druckzyklen, die in der Dosierkammer (14) in einer vordefinierten Dosierkonfiguration untergebracht ist, wobei die Vorrichtung eine Pulvereinlassöffnung (17) und ein Pulverauslassrohr (40) umfasst und die Dosiervorrichtung (13) geeignet ist, Mengen einer vorbestimmten Menge Pulver (11) durch das Auslassrohr (40) in Behälter (41) abzugeben; die Vorrichtung umfasst außerdem eine Zufuhreinheit (19) für die Pulver (11), die durch die Einlassöffnung (17) der Dosiervorrichtung (13) zugeführt werden sollen, und ist **dadurch gekennzeichnet, dass** sie eine Vakuumpumpe (55) umfasst, die mit der Dosierkammer (14) in Verbindung steht, und eine Zufuhreinheit (23) zum Zuführen eines Desinfektions-/Sterilisationsfluids, die direkt mit der Dosiervorrichtung verbunden ist, wobei die Zufuhreinheit (23) zum Zuführen eines Desinfektions-/Sterilisationsfluids so konfiguriert ist, dass sie das Desinfektions-/Sterilisationsfluid durch die Einlassöffnung (17; 17') in die Dosiervorrichtung (13) zuführt und dabei die Dosiervorrichtung (13) in der Dosierkonfiguration innerhalb der Dosierkammer (14) angeordnet ist, und wobei die Vakuumpumpe (55) so konfiguriert ist, dass sie die Dosierkammer (14) auf einen Druck bringt, der niedriger ist als der atmosphärische Druck, bevor das Desinfektions-/Sterilisationsfluid zugeführt wird, und dadurch, dass die Dosiervorrichtung (13) eine rotierende Volumen-Dosiervorrichtung (13) ist.

11. Dosiervorrichtung nach Anspruch 10, **dadurch gekennzeichnet, dass** sie eine Waschkammer (15) umfasst, in der mindestens eine Reinigungsvorrichtung (46) zum Zuführen eines Waschfluids installiert ist, und die so konfiguriert ist, dass sie während eines Waschschrittes mindestens die Dosiervorrichtung (13) aufnimmt, wobei die Dosierkammer (14) und die Waschkammer (15) nebeneinander liegen, normalerweise getrennt sind und durch bewegliche Trennmittel (33), die eine Durchgangsöffnung (49) abtrennen, miteinander in Verbindung stehen.

12. Dosiervorrichtung nach Anspruch 10, **dadurch gekennzeichnet, dass** sie mindestens eine Reinigungsvorrichtung (46) umfasst, die in der Dosierkammer (14) installiert ist und dazu konfiguriert ist, während eines Waschschritts ein Waschfluid in die Dosierkammer (14) zuzuführen.

13. Dosiervorrichtung nach Anspruch 10, **dadurch gekennzeichnet, dass** sie eine der Dosiervorrichtung direkt zugeordnete Abgabeeinheit (27) für ein Waschfluid aufweist.

14. Dosiervorrichtung nach einem der Ansprüche 10 bis 13, **dadurch gekennzeichnet, dass** die Zufuhreinheit (19) für die Pulver und die Abgabeeinheit (23) für ein Desinfektions-/Sterilisationsfluid Rohre (20, 25) umfassen, die in Fluidverbindung mit einer einzigen Sammeleinheit (22) stehen.

## Revendications

1. Méthode de désinfection d'un appareil de dosage de poudres (11), comprenant une chambre de dosage (14) et un dispositif volumétrique rotatif de dosage (13) pour poudres (11) avec des cycles de vide/pression, hébergé dans ladite chambre de dosage (14) dans une configuration de dosage prédéfinie, dans laquelle ledit appareil de dosage comprend une ouverture d'entrée de poudre (17) et un tuyau de sortie (40) pour poudre (11), ledit dispositif de dosage (13) étant adapté pour délivrer des doses d'une quantité prédéterminée de poudre (11) à travers ledit tuyau de sortie (40) à l'intérieur des conteneurs (41), le remplissage d'un nombre prédéfini de conteneurs (41) définissant un cycle de dosage de poudre; ladite désinfection étant effectuée après qu'un cycle de dosage de poudre a été terminé et comprenant une étape de lavage pour laver ledit dispositif de dosage (13) et une étape subséquente de désinfection/stérilisation effectuée in situ pour désinfecter/stériliser ledit dispositif de dosage (13) qui est maintenu dans ladite configuration de dosage à l'intérieur de ladite chambre de dosage (14) pendant l'étape de désinfection/stérilisation afin que, avant de réaliser un nouveau cycle de dosage de poudre, aucune manipulation ou déplacement dudit dispositif de dosage (13) ne soit prévue après ladite étape de désinfection/stérilisation, ladite méthode étant **caractérisée en ce que** ladite étape de désinfection/stérilisation comprend une première sous-étape dans laquelle ladite chambre de dosage (14) est amenée à une pression inférieure à 1a pression atmosphérique et une sous-étape subséquente dans laquelle, par ladite ouverture d'entrée (17; 17'), un fluide désinfectant/stérilisant (vapeur) est introduit à l'intérieur dudit dispositif de dosage (13), tandis que ladite chambre de dosage (14) est maintenue à ladite pression inférieure à la pression atmosphérique.

2. Méthode selon la revendication 1, **caractérisée en ce que** ladite étape de lavage prévoit d'introduire un fluide de lavage à l'intérieur de ladite chambre de dosage (14) par ladite ouverture d'entrée (17; 17') et de remplir ladite chambre de dosage (14) avec ledit fluide de lavage, et est suivie d'une étape subséquente de drainage du fluide de lavage hors de ladite chambre de dosage (14).

3. Méthode selon la revendication 1, **caractérisée en ce que** ladite étape de lavage prévoit de laisser ledit dispositif de dosage (13) dans ladite configuration de dosage prédéfinie à l'intérieur de ladite chambre de dosage (14), dans ladite étape de lavage il est prévu d'actionner un dispositif de nettoyage (46) installé dans ladite chambre de dosage (14), capable de délivrer un fluide de lavage dirigé en particulier vers ledit dispositif de dosage (13).

4. Méthode selon toute revendication précédente, **caractérisée en ce que** pendant ladite première sous-étape dans laquelle ladite chambre de dosage (14) est amenée à une pression inférieure à la pression atmosphérique il est prévu de créer un vide à l'intérieur de la chambre de dosage (14) au moyen d'une pompe à vide (55) connectée au tuyau de sortie (40) de la poudre (11), le vide étant créé après que l'ouverture d'entrée de poudre (17) a été fermée.

5. Méthode selon toute revendication précédente, **caractérisée en ce que**, dans ladite première sous-étape, il est prévu de porter ladite chambre de dosage (14) à une pression comprise entre -1 bar et -0.5 bar, notamment égale à environ -0.7 bar.

6. Méthode selon toute revendication précédente, **caractérisée en ce que** ladite étape de désinfection/stérilisation se déroule pendant une période de temps prédéfinie en cycles de livraison du fluide désinfectant/stérilisant à l'intérieur de ladite chambre de dosage (14), et donc à l'intérieur dudit dispositif de dosage (13), alternant avec des cycles de surpression au cours desquels la pression dans la chambre de dosage (14) est augmentée à une valeur supérieure à la pression atmosphérique pendant une période de temps prédéfinie.

7. Méthode selon la revendication 6, **caractérisée en ce que** lesdits cycles de surpression prévoient de porter ladite chambre de dosage (14) à une pression comprise entre 1.2 et 1.8 bar, notamment égale à environ 1.5 bar.

8. Méthode selon toute revendication précédente, **caractérisée en ce que**, après ladite étape de désinfection/stérilisation, elle comprend une étape de séchage pour sécher ladite chambre de dosage (14) et ledit dispositif de dosage (13).

9. Méthode pour le fonctionnement d'un appareil de dosage de poudres (11), comprenant une chambre de dosage (14) et un dispositif volumétrique rotatif de dosage (13) pour poudres (11) avec des cycles de vide/pression, hébergé dans ladite chambre de dosage (14) dans une configuration de dosage prédéfinie, dans laquelle ledit dispositif de dosage comprend une ouverture d'entrée de poudre (17) et un tuyau de sortie (40) pour poudre, ledit dispositif de dosage (13) étant également adapté pour délivrer des doses d'une quantité prédéterminée de poudre (11) à travers ledit tuyau de sortie (40) à l'intérieur de conteneurs (41), la remplissage d'un nombre prédéfini de conteneurs (41) définissant un cycle de dosage de poudre; ladite méthode comprenant l'accomplissement d'un cycle de dosage des poudres (11) au moyen de la distribution desdites doses de poudre (11) à l'intérieur desdits conteneurs (41), et un cycle subséquent de désinfection comprenant une étape de lavage pour laver ledit dispositif de dosage (13) et une étape subséquente de désinfection/stérilisation pour désinfecter/stériliser ledit dispositif de dosage (13) selon la méthode décrite dans toutes revendications de 1 à 8.

10. Appareil de dosage de poudres, comprenant une chambre de dosage (14) et un dispositif volumétrique de dosage (13) pour poudres (11) avec des cycles de vide/pression hébergé dans ladite chambre de dosage (14) dans une configuration de dosage prédéfinie, dans laquelle ledit appareil comprend une ouverture d'entrée de poudre (17) et un tuyau de sortie de poudre (40), et ledit dispositif de dosage (13) est adapté pour délivrer des quantités d'une quantité prédéterminée de poudre (11) à travers ledit tuyau de sortie (40) à l'intérieur des conteneurs (41); ledit appareil comprend également une unité d'alimentation (19) des poudres (11) à alimenter dans ledit dispositif de dosage (13) par ladite ouverture d'entrée (17), une pompe (55) communiquant avec ladite chambre de dosage (14) et une unité de distribution (23) pour délivrer un fluide désinfectant/stérilisant directement associé à l'appareil de dosage, ladite unité de distribution (23) pour délivrer un fluide désinfectant/stérilisant étant configurée pour délivrer ledit fluide désinfectant/stérilisant à l'intérieur dudit dispositif de dosage (13) par ladite ouverture d'entrée (17; 17'), maintenant ledit dispositif de dosage (13) disposé dans ladite configuration de dosage à l'intérieur de ladite chambre de dosage (14), ledit appareil étant **caractérisé en ce que** ladite pompe est une pompe à vide (55) qui est configurée pour amener ladite chambre de dosage (14) à une pression inférieure à la pression atmosphérique avant que ledit fluide désinfectant/stérilisant ne soit délivré et **en ce que** ledit dispositif de dosage (13) est un dispositif de dosage rotatif volumétrique (13).

11. Appareil de dosage selon la revendication 10, **caractérisé en ce qu'**il comprend une chambre de lavage (15) dans laquelle au moins un dispositif de nettoyage (46) est installé pour délivrer un fluide de lavage, et configuré pour héberger au moins le dispositif de dosage (13) pendant une étape de lavage, ladite chambre de dosage (14) et ladite chambre de lavage (15) étant adjacentes et normalement séparées, et mises en communication l'une avec l'autre au moyen de moyens de séparation mobiles (33) qui interceptent une ouverture de passage (49).

12. Appareil de dosage selon la revendication 10, **caractérisé en ce qu'**il comprend au moins un dispositif de nettoyage (46) installé dans ladite chambre de dosage (14) et configuré pour délivrer un fluide de lavage à l'intérieur de ladite chambre de dosage (14) pendant une étape de lavage.

13. Appareil de dosage selon la revendication 10, **caractérisé en ce qu'**il comprend une unité de distribution (27) d'un fluide de lavage directement associée à l'appareil de dosage.

14. Appareil de dosage selon toute revendication de 10 à 13, **caractérisé en ce que** ladite unité d'alimentation (19) des poudres et ladite unité de distribution (23) d'un fluide désinfectant/stérilisant comprennent des tuyaux (20, 25) en communication fluidique avec un seul collecteur (22).
